# EUROPEAN PATENT APPLICATION

(11) **EP 4 403 549 A1**
(43) Date of publication of application: **24.07.2024**
(21) Application number: 22869790.0
(22) Date of filing: 26.08.2022
(51) Int. Cl.: C07C 323/09, A61K 47/68, A61P 35/00, C07C 323/62

(54) **LINKER COMPOUND AND COMPLEX COMPOUND CONTAINING SAME**

(30) Priority: 17.09.2021 JP 2021152092
(71) Applicant: Tohoku University, Sendai-shi, Miyagi 980-8577 (JP)
(72) Inventor: WADA, Takehiko, Sendai-shi, Miyagi 980-8577 (JP)
(74) Representative: Cabinet Becker et Associés
(86) International application number: PCT/JP2022/032220
(87) International publication number: WO 2023/042643

(57) **Abstract**

There are provided a linker compound, for which a glutathione concentration at which a disulfide bond dissociates can be designed by molecular design, and which can accurately identify a cancer cell, is stable outside a target cell, and can effectively release a drug compound in the target cell, and a complex compound containing the same. The linker compound is used for a complex compound containing a drug compound and has a structure represented by the following Formula (1). (In Formula (1), R is a compound containing hydrogen, carbon, oxygen, or a halogen element, or a derivative thereof.

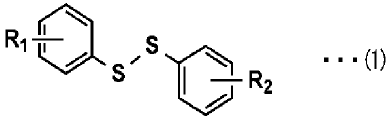

## Description

### Technical Field

The present invention relates to a linker compound used to form a complex compound in which a drug compound used for treatment is complexed with another compound, and a complex compound using the same.

This application claims priority based on Japanese Patent Application No. 2021-152092 filed in Japan on September 17, 2021, the contents of which are incorporated herein.

### Background Art

Cancer is a disease that ranks high in both morbidity and mortality in Japan and the world, and the establishment of a treatment method therefore is one of the most important problems. In recent years, a biopharmaceutical having a relatively high molecular weight, which is produced from a low-molecular weight drug by using a gene recombination technique, has been developed as a drug against cancer. As a typical example of the biopharmaceutical, an antibody drug has been developed using the distinguishability of an antibody. The antibody drug has the advantage of higher target specificity and fewer side effects as compared with the low-molecular-weight drug. Thus, the antibody drug is expected to be applicable to a method for treating cancer by specifically acting on cancer cells.

The antibody drug can be developed in a short period of time by utilizing an existing drug and antibody, is expected to be developed by a drug development strategy with a relatively low development cost and a high probability of success, and is also expected in terms of potential for development of business and market in drug discovery.

On the other hand, the antibody drug generally has many problems such as complicated production steps, high production and development costs, a drug made to have a high molecular weight, and antigenicity. Furthermore, due to the high molecular weight or the like, cell membrane permeability is extremely low, and thus, a target may be limited to an extracellular protein of a cell surface layer or the like.

To solve this problem, a technique has been developed in which an antibody and a drug are bound to each other via a linker compound to form a complex (antibody-drug complex), and the linker compound is provided with a dissociable function to allow the drug to exert its function as a simple substance separate from the antibody.

For example, as compound applicable to the antibody drug, Patent Document 1 discloses a drug-antibody conjugate compound in which an antibody and a drug are bound via a linker compound.

This drug-antibody conjugate compound is intended to form a complex of a pyrrolobenzodiazepine prodrug monomer (drug), an antibody, and a linker compound by forming a disulfide bond with cysteine in the Fc region of the antibody using the linker compound having a sulfide group.

Non-Patent Document 1 discloses an alkane-type disulfide linker as a linker for binding an antibody and a drug.

This linker cleaves the disulfide linker in a glutathione concentration-dependent manner, and the cleavage is to be evaluated using FRET.

Non-Patent Document 2 discloses a dithiobenzylurethane linker as a linker for binding an antibody and a drug.

This linker cleaves the disulfide linker in a glutathione concentration-dependent manner.

### Citation List

### Patent Document

Patent Document 1: JP 2019-534238 T

### Non-Patent Literature

Non-Patent Document 1: Scales et al., Bioconjugate Chem 30 (2019) 3046-3056
Non-Patent Document 2: Zheng et al., ChemMedChem 14, (2019) 1196-1203

### Summary of Invention

### Technical Problem

In the techniques of Patent Document 1 and Non-Patent Documents 1 and 2, an antibody and a compound constituting a drug are bound to each other via a disulfide bond of a linker compound, and the antibody and the compound constituting the drug are cleaved depending on the glutathione concentration, whereby the drug can exert a therapeutic function. It is generally said that the concentration of glutathione is 5 to 25 µM in blood, and 0.4 mM or more and less than 10 mM in a normal cell. For example, in Patent Document 1, a range in which the glutathione concentration is more than three digits (on the order of mM) is identified as an intracellular concentration. When an antibody-drug complex is administered into the blood, the complex remains formed because the glutathione concentration is low (on the order of µM). When the antibody-drug complex is transferred into a cell, the glutathione concentration becomes high (on the order of mM), the disulfide bond of the linker compound is cleaved, and the complex of the antibody and the compound constituting the drug is dissociated, allowing the drug to exert its efficacy in the cell.

However, in the techniques of Patent Document 1 and Non-Patent Documents 1 and 2, the linker compound can only dissociate a complex of an antibody and a compound constituting a drug only at a certain concentration depending on a structure of each compound.

For example, in the antibody-drug complex of Patent Document 1, the complex dissociates when the concentration of glutathione is in the order of mM. Thus, when the complex is transferred into a cell, regardless of whether the cell is a normal cell or a cancer cell, the drug exerts its effect in the cell. Accordingly, the drug will also dissociate even in the normal cell, and thus, the drug may act on an undesired cell to cause a side effect.

In this regard, it is known that a cancer cell has a glutathione concentration of 10 to 100 times higher than that of a normal cell, averaging about 10 to 200 mM on average. The present inventors have considered that a cancer cell and a normal cell can be distinguished from each other by the difference in glutathione concentration between the cancer cell and the normal cell. However, for example, in the linker compound disclosed in Patent Document 1, due to the structure of the linker compound, it is possible to distinguish cells when their glutathione concentrations are in the order of µM and mM, respectively, but it is impossible to identify a more precise concentration difference such as the glutathione concentration difference between a cancer cell and a normal cell to cleave the linker compound.

The disulfide bond linker used in Non-Patent Documents 1 and 2 is an alkane-type linker based on a thiol group such as cysteine. In the alkane-type linker, the ease of dissociation and binding can be adjusted by adjusting steric hindrance of an S atom, but it is difficult to provide high selectivity according to various glutathione concentrations.

Furthermore, the glutathione concentration in a cancer cell varies depending on the type of cell and the state of cancer. Thus, even when a linker compound that dissociates at a certain glutathione concentration is used, it may be insufficient for use in the treatment of various cancer cells and states of cancer.

In view of these circumstances, the present inventors have considered that various cancer cells can be identified, a target cell can be accurately selected, and an antibody-drug complex can be used for effective cancer treatment, by using a multifunctional linker compound having a higher degree of freedom of functions by design. Specifically, the present inventors have considered that if the glutathione concentration at which the disulfide bond dissociates can be adjusted by molecular design, a multifunctional linker compound capable of identifying various cancer cells and states of cancer by the glutathione concentration can be obtained, and pursued further studies on the linker compound.

The present invention has been made in view of the circumstances as described above, and the present invention is directed to providing a linker compound, for which a glutathione concentration at which a disulfide bond dissociates can be designed by molecular design, and which can accurately identify a cancer cell, is stable outside a target cell, and can effectively release a drug compound in the target cell, and a complex compound containing the same.

### Solution to Problem

To solve the problems described above, the present invention has the following aspects.
[1] A linker compound for use in a complex compound containing a drug compound, the linker compound having a structure represented by the following formula (1): 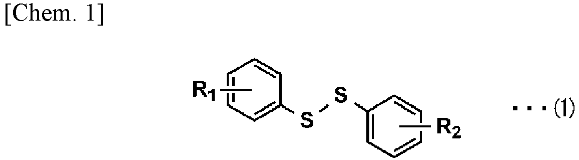 where R₁ and R₂ are each a compound containing hydrogen, carbon, oxygen, nitrogen, or a halogen element, or a derivative thereof.
[2] The linker compound, wherein, in the formula (1), R₁ and R₂ are each a halogen element, a hydrocarbon group, an oxide group, or a nitride group.
[3] The linker compound, wherein, in the formula (1), R₁ and R₂ are each a halogen element, a methyl group, a t-butyl group, a methoxy group, a nitro group, a carboxyl group, a cyano group, or a dimethylamino group.
[4] The linker compound, wherein, in the formula (1), R₁ and R₂ are each a nitro group, fluorine, or a carboxyl group.
[5] The linker compound, wherein, in the formula (1), the S-S bond is cleaved at a glutathione concentration of 10 to 200 mM.
[6] The linker compound, wherein the complex compound is the antibody-drug complex compound in which the drug compound is complexed with a drug compound-transporting peptide via the linker compound.
[7] The linker compound according to claim 6, wherein the drug compound-transporting peptide is selected from the group consisting of an antibody and a cell membrane permeable peptide.
[8] A complex compound including the linker compound.
[9] The complex compound, being an antibody-drug complex compound in which the drug compound is complexed with an antibody via the linker compound.
[10] The complex compound, wherein the drug compound is an apoptosis fusion peptide.
[11] The complex compound, wherein the drug compound is a nucleic acid.
[12] The complex compound, being used against a cancer cell.

### Advantageous Effects of Invention

According to the present invention, there are obtained a linker compound, for which a glutathione concentration at which a disulfide bond dissociates can be designed by molecular design, and which can accurately identify a cancer cell, is stable outside a target cell, and can effectively release a drug compound in the target cell, and a complex compound containing the same.

### Brief Description of Drawings

FIG. 1 is a graph showing NMR spectra of compounds used in Test Example 1.
FIG. 2 is a graph of comparison of the NMR spectra of the compounds of FIG. 1 in an overlapping manner.
FIG. 3 is a graph showing NMR spectra of compounds used in Comparative Example 1.
FIG. 4 is a graph showing an NMR spectrum of a compound used in Test Example 2.
FIG. 5 is a partially enlarged view of FIG. 4.
FIG. 6 is a graph showing a result obtained by plotting a change in absorption intensity at 242 nm of FIG. 4 with respect to time.
FIG. 7 is a graph showing NMR spectra of some compounds used in Test Example 3.
FIG. 8 is a graph showing NMR spectra of some compounds used in Test Example 3.
FIG. 9 is a graph showing NMR spectra of some compounds used in Test Example 3.
FIG. 10 is a graph showing NMR spectra of some compounds used in Test Example 3.
FIG. 11 is a graph showing NMR spectra of compounds used in Test Example 4.
FIG. 12 is a graph showing NMR spectra of other compounds used in Test Example 3.
FIG. 13 is a partially enlarged view of FIG. 12.
FIG. 14 is another partially enlarged view of FIG. 12.
FIG. 15 is a confocal microscopic view of fluorescence development of cells used in Test Example 9.

### Description of Embodiments

Hereinafter, a linker compound and a complex compound according to the present invention will be described with reference to an embodiment. However, the present invention is not limited to the embodiment described below.

### Linker Compound

A linker compound of the present embodiment is a linker compound used for a complex compound containing a drug compound, and has a structure represented by the following formula (1). 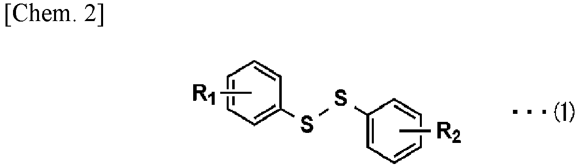 (In the formula (1), R₁ and R₂ are each a compound containing hydrogen, carbon, oxygen, or a halogen element, or a derivative thereof.)

The term "linker compound" refers to a compound that is used to bind a plurality of other compounds to form a complex compound with the other compounds. The linker compound is bonded to another compound mainly by substitution on the benzene ring or for R₁ or R₂ of the formula (1) to form a complex compound.

The linker compound is used for a complex compound containing a drug compound. That is, at least one of a plurality of compounds which are directly or indirectly bonded to the linker compound to form the complex compound is a drug compound.

The term "drug compound" broadly refers to a compound that acts as a drug or a compound that exerts a therapeutic effect. Preferably, the drug compound is used in the treatment of cancer. In this case, the complex compound is preferably used against a cancer cell. In the linker compound of the present embodiment, a linker binding portion dissociates depending on a glutathione concentration, and thus, the drug compound is separated from the complex compound when introduced into a cancer cell having a high glutathione concentration, and is suitable for functioning as a drug.

In the formula (1), R₁ and R₂ are each a substituent in the aromatic ring in the formula. R₁ and R₂ each may be a substituent at any position in the aromatic ring. In the formula (1), R₁ is a substituent on one aromatic ring and R₂ is a substituent on the other aromatic ring, but each of R₁ and R₂ may be a plurality of substituents and may be a substituent at a plurality of positions on the aromatic ring. In addition, R₁, R₂, and a plurality of substituents which may be contained therein may be the same substituent or different substituents. The substituent may be located at any position of an ortho position, a meta position or a para position with respect to a position to which S is bonded on the aromatic ring.

The present inventors have considered that, for a linker compound having an S-S bond, a condition under which the S-S bond dissociates, specifically a reducing condition such as the glutathione concentration, can be adjusted by adjusting an electron density of the S atom of the S-S bond.

A thiol-disulfide exchange reaction between the S atom and the S-S bond is controlled by an acidity (pKa) of thiol, an electronic environment of the S atom, steric hindrance, entropy, or the like. For this reason, it is necessary to change the above factors to control reactivity of the disulfide linker with GSH.

As a linker candidate compound, it was expected to control the environment of the S atom and control the reactivity with GSH by adjusting the electronic environment in the aromatic ring by the substituent (-R) of the aromatic ring bonded to or adjacent to the S atom. Then, at first, attention was paid to use of benzylthiol derivatives (formula (10) and formula (11) described below) that can be synthesized from commercially available isophthalic acid. However, with the benzylthiol derivatives, it was impossible to obtain a difference in electron density by proton NMR depending on presence or absence of a substituent, and designing as a linker compound was considered to be difficult.

Then, the present inventors examined use of a thiophenol derivative (formula (1)) having a structure in which the S atom is closer to the aromatic ring than in the benzylthiol derivative and which is easily affected by the aromatic ring and the electron density of the substituent, that is, the S atom is directly bonded to the aromatic ring. As a result, it has been found that in this compound, the electron density on the S atom and the reactivity with GSH can be controlled by the substituent on the aromatic ring.

In the formula (1), each of R₁ and R₂ is preferably a halogen element, a hydrocarbon group, an oxide group, or a nitride group. In the formula (1), each of R₁ and R₂ is also preferably a halogen element, a methyl group, a t-butyl group, a methoxy group, a nitro group, a carboxyl group, a cyano group, or a dimethylamino group.

In the formula (1), the electron density of the S atom in the formula (1) can be variously designed by selecting R₁ and R₂ from the element, substituents, or compounds. The condition under which S-S in the above formula (1) disassociates can be controlled by the design.

In the formula (1), it is also preferable that R₁ and R₂ are each a nitro group, fluorine, or a carboxyl group.

When R₁ and R₂ are each selected from the element, substituents, or compounds in the formula (1), the electron density of the S atom in the formula (1) can be designed to provide a structure suitable for selective dissociation depending on the glutathione concentration in a cancer cell.

Specifically, when each of R₁ and R₂ is an ortho-coordinated carboxyl group, it is possible to obtain a structure in which 80% or more does not dissociate in normal cells one day after administration and nearly 100% dissociates in a cancer cell model having a glutathione concentration of 20 mM one day after administration.

In addition, in a case where both R₁ and R₂ are ortho-coordinated carboxyl groups and meta-coordinated fluorine elements, it is possible to obtain a structure in which 70% or more dissociates 100 minutes after administration to a cancer cell model having a glutathione concentration of 20 mM.

In addition, in a case where both R₁ and R₂ are ortho-coordinated carboxyl groups and meta-coordinated fluorine elements, it is possible to obtain a structure in which 70% or more dissociates 100 minutes after administration to a cancer cell model having a glutathione concentration of 20 mM.

In addition, in a case where both R₁ and R₂ are meta-coordinated carboxyl groups and para-coordinated nitro group elements, it is possible to obtain a structure in which 80% or more dissociates 100 minutes after administration to a cancer cell model having a glutathione concentration of 20 mM.

In the formula (1), it is also preferable that the S-S bond is cleaved at a glutathione concentration of 10 to 200 mM. With this configuration, the S-S bond can be cleaved at glutathione concentrations in various cancer cells. In general, the magnitude (order) of the glutathione concentration in a tissue is considered to be about 1 to 10 µM in blood, about 1 to 10 mM in a normal cell, and about 10 to 200 mM in a cancer cell in terms of molar concentration. In particular, on average, the concentration may be in a range of 5 to 25 µM in blood, 0.4 mM or more and less than 10 mM in a normal cell, and 10 mM or more and 200 mM or less in a cancer cell. Accordingly, it is possible to design the linker compound in such a manner that the linker compound and a linker portion of the complex compound containing the same are cleaved at a glutathione concentration of a cancer cell under a specific condition included in 10 to 200 mM by configuring the linker compound in such a manner that the S-S bond is cleaved at a glutathione concentration of 10 to 200 mM.

Specifically, as described above, the structure of the compound of the formula (1), more specifically, the structures of R₁ and R₂ and the substitution position on the aromatic ring are designed, whereby it is possible to design the electron density of the S atom and control the glutathione concentration at which the S-S bond is cleaved.

The linker compound of the present embodiment can be widely used to form a complex compound with a drug compound and another compound.

Specifically, as described below, the linker compound can be used for an antibody-drug complex compound in which the drug compound is complexed with an antibody via the linker compound.

### Complex Compound

A complex compound of the present embodiment includes the linker compound. That is, the complex compound includes a drug compound and the linker compound. In particular, the complex compound includes a compound in which a drug compound is complexed with another compound via the linker compound.

The complex compound is preferably an antibody-drug complex compound in which the drug compound is complexed with a drug compound-transporting peptide via the linker compound.

As used herein, the term "drug compound-transporting peptide" broadly refers to a peptide which is complexed with a drug compound and has a function of directing the drug compound to a target. The term "peptide" as used herein refers to a combination of a plurality of amino acids and broadly refers to a combination of several amino acids to a large polypeptide (so-called protein). The term "target" refers to, for example, a specific cell or a specific position in a cell. The term "specific cell" refers to a cell having a specific property such as a cancer cell, and the term "specific position in the cell" refers to the inside of the cell, the surface of the cell, or the like.

It is also preferable that the drug compound-transporting peptide is selected from the group consisting of antibodies and cell membrane permeable peptides. Examples of these effects include a function of guiding a desired drug to a desired site in such a manner that an antibody delivers a drug compound to a target cancer cell or a cell membrane permeable peptide such as a linker peptide delivers a drug compound into a cell.

The cell membrane permeable peptide is also preferably a linker peptide (peptide linker) that binds the linker compound to another compound.

As the cell membrane permeable peptide which is a linker peptide, for example, an arginine linker having an oligoarginine sequence in which a plurality of arginines are bonded can be used. A peptide having the oligoarginine sequence or an arginine-rich peptide promotes uptake into cells, and thus, such a peptide is used as an arginine linker to bind to another compound, which allows the compound to be taken into the cytoplasm from the outside the cells.

It is also preferable that the complex compound is an antibody-drug complex compound in which the drug compound is complexed with an antibody via the linker compound.

The antibody-drug complex compound can be used as one kind of antibody drug. That is, the antibody-drug complex compound has high selectivity by specifically binding to a target (antigen) of the antibody. Furthermore, in the antibody-drug complex compound, the linker compound is cleaved depending on an intracellular environment, for example, a reducing environment (glutathione concentration) to release the drug compound from the antibody-drug complex compound. Thus, the combination of the antibody and the linker compound can exert particularly high selectivity.

It is also preferable that the drug compound is a so-called drug, an amino acid, an oligopeptide, a protein drug, or a nucleic acid drug, which has efficacy.

The oligopeptide of the drug compound is also preferably an apoptosis-inducing peptide. Examples of the apoptosis-inducing peptide include PAD/kla (KLAKLAK)₂. An apoptosis fusion peptide induces apoptosis of a cell, and thus can kill a cancer cell for therapeutic use. The linker compound of the present embodiment and the complex compound including the same can specifically target a cancer cell, and further a cancer cell under a specific condition, and thus can be used for treatment for killing a specific cancer cell.

The drug compound of the present embodiment is also preferably a nucleic acid. The nucleic acid of the drug compound can be selected from those used as nucleic acid drugs.

The nucleic acid drug is a therapeutic agent that targets a chromosomal DNA, various functional RNAs, and further a protein, and exerts efficacy by function control using a nucleic acid derivative. With respect to a target disease, particularly in an antisense method targeting a functional RNA, it is possible to design a variety of nucleic acid drugs based on the sequence, expression timing, expression site, and the like, and there are many potential applicable diseases. On the other hand, in a nucleic acid drug in the related art, it has been reported that a derivative having high affinity also forms a complex with a non-target RNA having a sequence similar to that of the target and causes a side effect called an off-target effect, which is a serious problem.

When a nucleic acid drug is used as the drug compound of the present embodiment and the linker compound of the present embodiment is used to form a complex compound, the high selectivity of the linker compound can be obtained and a compound having selectivity can be selected as another compound constituting the complex compound. Thus, it is possible to obtain high selectivity for a specific target and to prevent a side effect.

Another compound can also be used as the compound used in the complex compound of the present embodiment. For example, the linker compound of the present embodiment is applicable as a linker with polyethylene glycol (PEG) or the like, which is frequently used as a general-purpose DDS system, and can also be used as a linker compound that binds a drug to a peptide-based vector, a sugar chain-based vector such as GalNAc, or a pilot molecule. It can also be used as a coupled linker with a deactivated molecule as a prodrug. The drug to be bonded to and/or loaded with the linker compound is not limited to the above-described compounds, and the linker compound can be applied to various drugs. Thus, the linker compound can be used for a complex compound obtained by complexing these drugs.

The complex compound of the present embodiment can appropriately select a target cell depending on a reducing condition, particularly a condition of the glutathione concentration. In particular, it is preferably used for a cancer cell. A cancer cell has a glutathione concentration in a specific range, and the complex compound of the present embodiment can specifically select a target.

### Action and Effect of Linker Compound and Complex Compound of Present Embodiment

As an action of the linker compound of the present embodiment, the linker compound has a structure represented by the formula (1), and a disulfide bond (S-S bond) in the structure of the formula (1) dissociates under a reducing condition. For example, it dissociates when the glutathione (GSH) concentration becomes high. The reducing condition for dissociation, such as the glutathione concentration, varies depending on the structure represented by the formula (1). For example, an oxidation-reduction potential of the disulfide bond varies depending on a coordination position and a structure of a substituent R on the aromatic ring in the formula (1). In other words, the condition under which the disulfide bond dissociates can be set by design of the substituent R on the aromatic ring.

By this action, the linker compound of the present embodiment identifies the glutathione concentration in the order of mM, specifically, the glutathione concentration within 10 to 200 mM, and dissociates when placed under a specific concentration condition. Thus, the linker compound does not dissociate under a specific concentration condition, for example, when introduced into blood or a normal cell having a glutathione concentration in the order of µM. On the other hand, the linker compound can dissociate when introduced into a target disease cell having a glutathione concentration above the specific concentration condition and a certain intracellular glutathione concentration.

The linker compound of the present embodiment has the action as described above, and thus it is possible to particularly accurately identify a cancer cell. Specifically, a condition under which the disulfide bond dissociates to dissociate the linker compound can be set in accordance with the difference in glutathione concentration between the normal cell and the cancer cell, or in accordance with the difference in glutathione concentration corresponding to the type of the cancer cell or the state of the cancer. Accordingly, it is possible to accurately identify the cancer cell and the type and state thereof, dissociate the linker compound, and release the drug compound in the cell. This results in a linker compound that is stable outside a target cell and can effectively release the drug compound in the target cell. That is, the linker composition of the present embodiment has designability and selectivity, and can be applied as a multifunctional linker exhibiting an advanced function.

In addition to the identification of a cancer cell and dissociation of a complex in a cell, the linker compound of the present embodiment can also dissociate by other glutathione concentrations or reducing conditions, and can be widely applied as a linker compound that dissociates under various designable conditions in various situations.

The complex compound of the present embodiment has a linker compound having the above-described action and effect. The complex compound of the present embodiment can be used in various situations where identification of a reduced state by the linker compound is applicable, for example, in drug delivery in the treatment of various diseases. The complex compound of the present embodiment can be widely used as a complex compound that can dissociate and release a drug compound depending on a glutathione concentration that can be variously designed in various situations, without being limited to discrimination between a normal cell and a cancer cell or presence or absence of dissociation in a cell.

Specifically, in a case where a compound having specific selectivity such as an antibody and a drug compound are bound to each other via a linker compound to form a complex compound, the complex compound can be stable in and efficiently introduced into a cell other than the target and can effectively release the drug in the target cytoplasm.

The complex compound of the present embodiment can be particularly suitably used for treatment of cancer.

For treatment of cancer, a drug delivery system (DDS) selective for cancer cells is required, which is capable of selective drug release in the vicinity of cancer cells, effective drug escape from endosomes, selective drug release in a cell environment characteristic of cancer cells, and the like.

In a case where a compound having specific selectivities such as an antibody and a drug compound are bound to each other via a linker compound to form a complex compound, the complex compound of the present embodiment can be widely applied to the DDS because a cancer cell selection system by the linker compound of the present embodiment, effective drug escape from endosome, and selective drug release in a cell environment characteristic of cancer cells are possible in addition to cell membrane permeability and in vivo circulation characteristics.

Although the embodiment of the present invention has been described above, the present invention is not limited to the above-described embodiment, and various modifications can be made.

### Examples

Hereinafter, the effects of the present invention will be made more apparent by Examples and Comparative Examples. Note that the present invention is not limited to the following Examples, and can be carried out with appropriate modifications without changing the gist of the invention.

### Test Example 1

### Substituent Effect of Thiophenol Derivative Model Compound: Proton NMR Measurement

Various examinations were conducted using a group of compounds represented by a formula (2) to a formula (9) as thiophenol derivatives. These thiophenol derivatives were commercially available, and the compounds represented by the formula (2) to the formula (6) were used as model compounds having various electron-withdrawing groups and electron-donating groups. In addition, the formula (7) to the formula (9) having a substituent (-COOH) were also used in various examinations. The substituent (-COOH) can be expected to be used in the subsequent condensation with a drug/transport site, and it was determined that the substituent (-COOH) was also appropriate in terms of increasing water solubility of highly hydrophobic disulfides. Actually, these compounds are soluble in water and also soluble in organic solvents such as THF, and thus, various examinations can be conducted in various solvents. The compound of the formula (9) is called an Ellman's reagent and is used as a reagent for the quantitative determination of thiol.

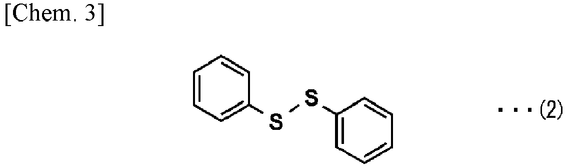

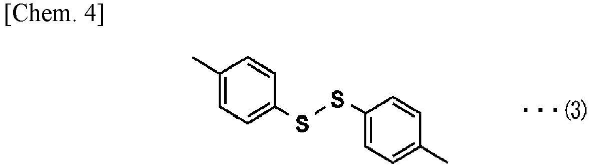

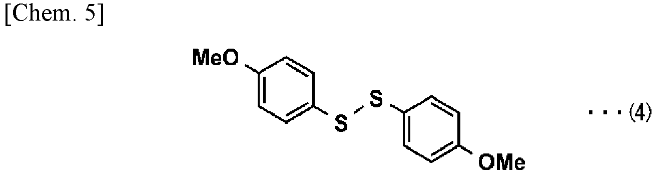

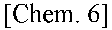

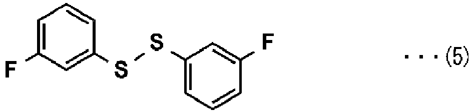

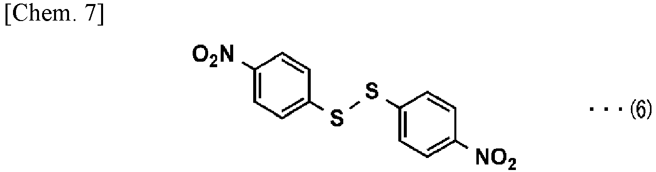

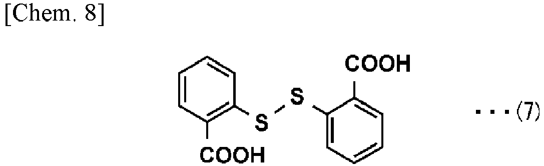

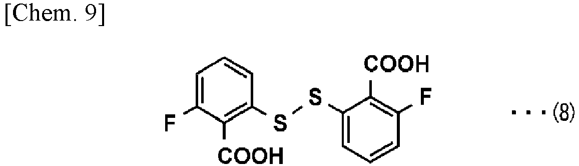

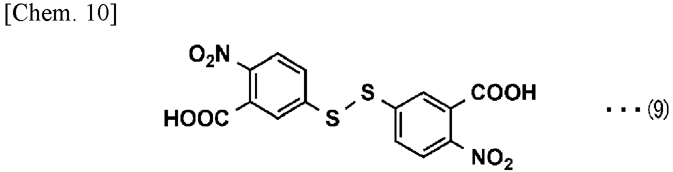

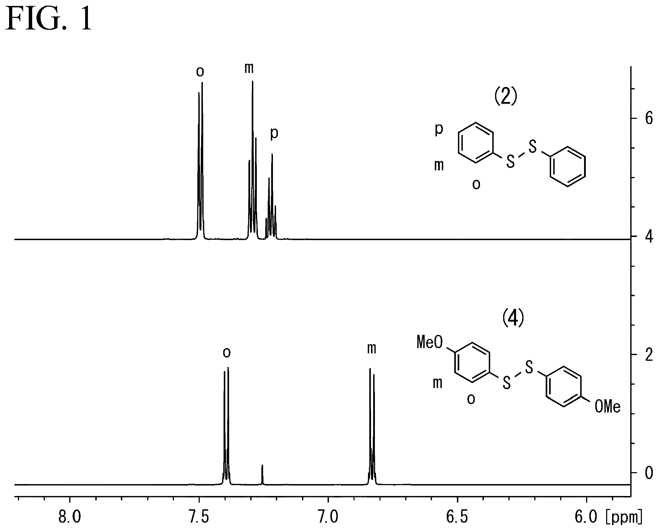

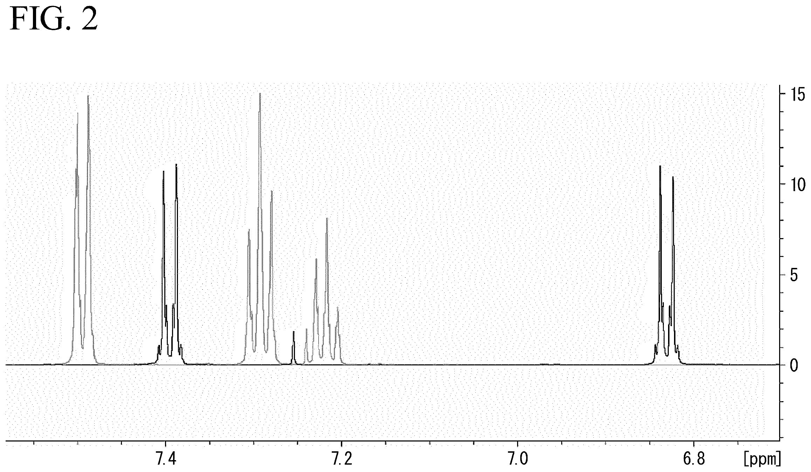

The influence of a substituent on an electron density on an S atom was confirmed by NMR measurement. 33S NMR and the like are also conceivable. However, 33S NMR has low sensitivity and a wide and broad peak, and thus, it was considered that useful information could not be obtained. Then, chemical shifts of protons at a benzyl position which is the root of the S atom were compared. FIG. 1 shows comparison of results of proton NMR spectra in CDCl3 for the compounds of the formula (2) and the formula (4). FIG. 2 shows a graph of comparison of the spectra of FIG. 1 in an overlapping manner.

Assignments and chemical shifts of peaks of the formula (2) that is unsubstituted and the formula (4) that is methoxy-substituted are as follows.
R = H 7.4933 ppm 4H (ortho position) R = H 7.2927 ppm 4H (meta position)
R = H 7.216 ppm 2H (para position)
R = OMe 7.4933 ppm 4H (ortho position) R = OMe 6.8304 ppm 4H (meta position)

From the result of the overlapped spectra, it can be confirmed that there is a large difference in chemical shift between the unsubstituted compound and the methoxy-substituted compound. From this result, it was suggested that in the thiophenol derivative disulfide linker, the electron density on the S atom and the reactivity with GSH were able to be controlled by the substituent on the aromatic ring.

### Comparative Example 1

### Substituent Effect of Benzylthiol Derivative Model Compound: Proton NMR Measurement

It was considered that a benzylthiol derivative condensed with a drug/transport module could be synthesized by a simple functional group conversion reaction from isophthalic acid, for which derivatives having various substituents are commercially available at a low cost. It was considered that the compound was useful as a linker compound if it could be controlled by a substituent on the aromatic ring, and an initial examination was conducted. In general, it is not known that an electron density can be controlled by a substituent on an aromatic ring in a reaction of the substituent at a benzyl position. Thus, the substituent effect of the benzylthiol derivative was compared between compounds represented by the following formula (10) and formula (11) using a model compound. Benzylthiol of the formula (10) and a compound of the formula (11) in which R = H of the compound of the formula (10) was substituted with a substituent OMe at two positions were used.

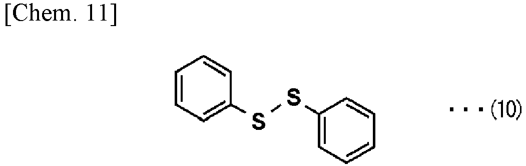

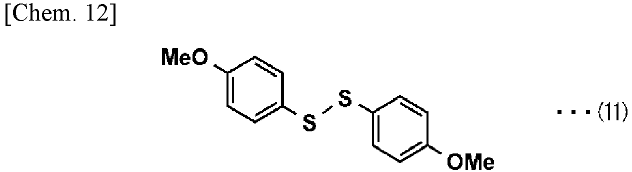

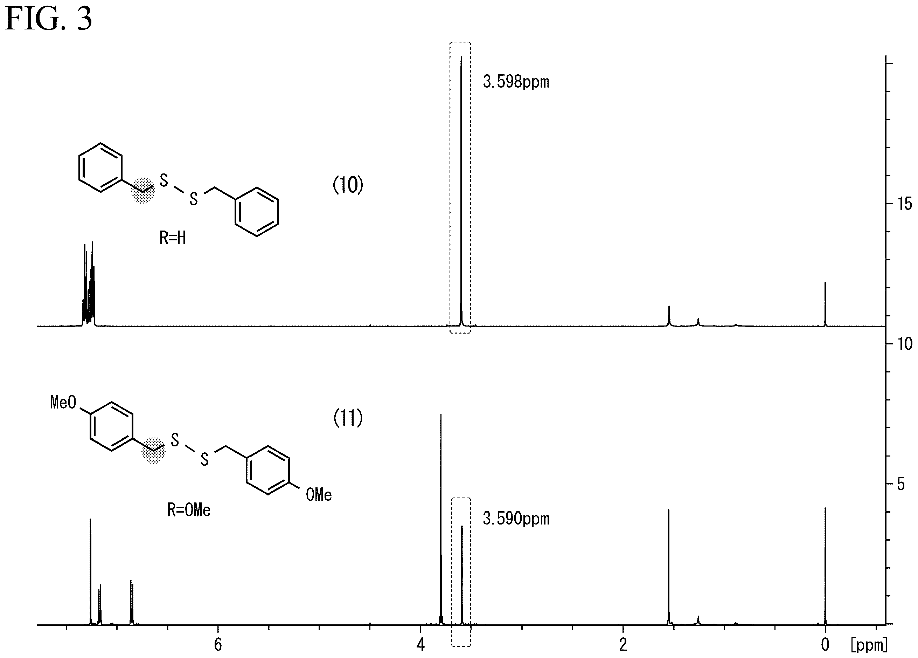

NMR measurement was conducted in the same manner as in Test Example 1. Comparison of spectral results of proton NMR in CDCl3 is shown in FIG. 3.

In the results of FIG. 3, when 2H at the benzyl position was compared between substituents (R = H, OMe).
R = H 3.598 ppm, and
R = OMe 3.590 ppm were obtained, and
no significant difference was able to be confirmed. From this result, it was determined that it was difficult to rationally design a disulfide linker compound having controllable reactivity with GSH by the substituent effect of an aromatic ring using a benzylthiol derivative. Thus, as in Test Example 1, a thiophenol derivative in which an S atom was more directly bonded to an aromatic ring was selected as a linker candidate compound, and various examinations were conducted.

### Test Example 2

### Examination of Reductive Cleavage Reaction Using UV Spectrum

The control of the electron density of the S atom by the substituent was expected from the above NMR results, and thus, a reductive cleavage reaction of the thiophenol-type disulfide linker was actually examined. First, a reaction of a thiophenol derivative model compound (compound of formula (2)) with a reducing agent LiBH₄ in an organic solvent THF was carried out as a model reaction (formula (12)) using a UV spectrum which can be easily examined, and a pseudo-first order reaction rate was examined.

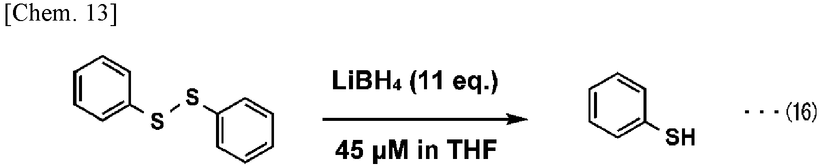

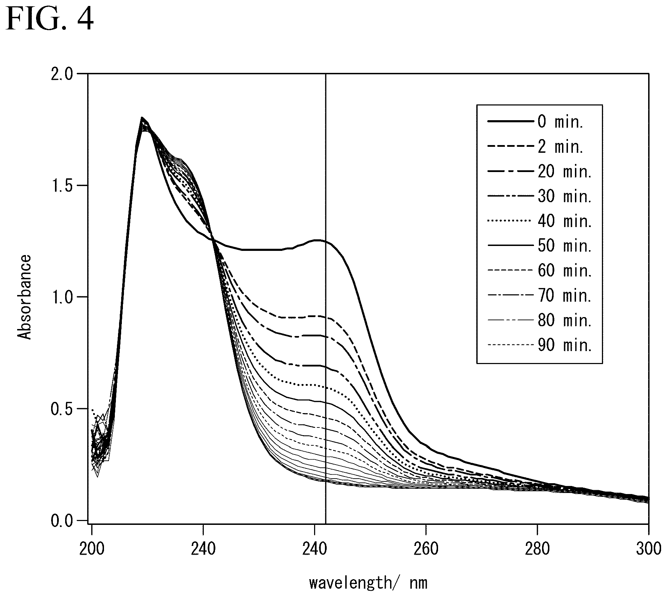

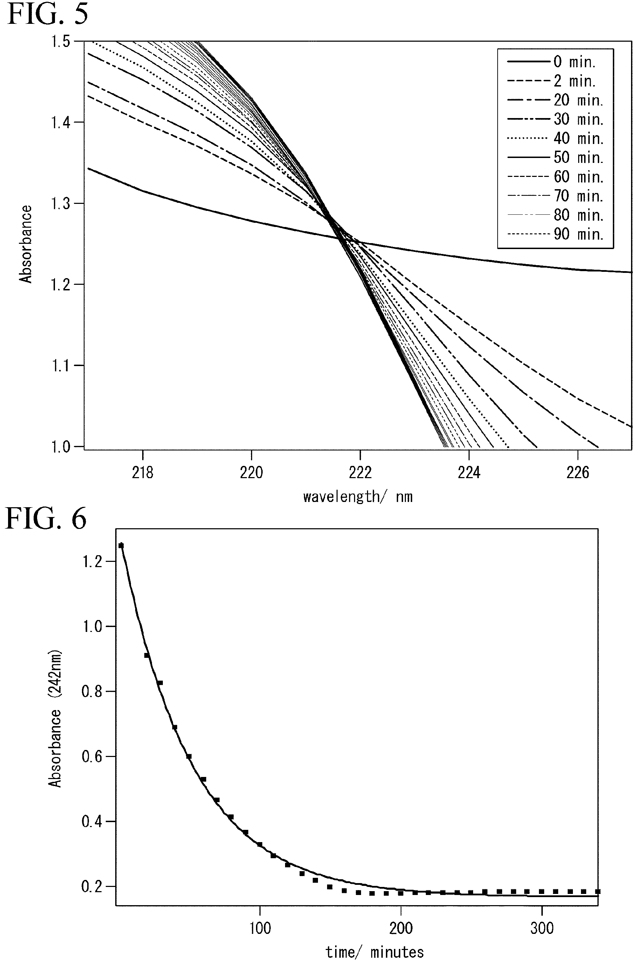

As the THF, THF available from Wako Pure Chemical Industries, Ltd., product code 207-17765, super dehydrated, and stabilizer-free was used, and as the cell, a cell of 10 mm × 10 mm was used. A reduction reaction of an oxidized form (disulfide) of the compound of the formula (2), which is an unsubstituted model compound, was carried out in the cell. The reaction was carried out using an excess amount (11 equivalents) of LiBH₄ relative to the oxidized form of the compound of the formula (2) (45 µM in THF). LiBH₄ was adjusted by diluting a commercially available THF 2M solution and used in the reaction. FIG. 4 shows changes in the UV spectrum with progress of the reaction. FIG. 5 shows a graph in which the absorption is 1.0 to 1.5 on the vertical axis and the wavelengths 216 to 228 nm on the horizontal axis in FIG. 4 are magnified.

The spectral results show an isosbestic point, and thus, it has been revealed that the model oxidized disulfide reacts to a reduced cleavage product in two state change. FIG. 6 shows a result obtained by plotting a change in absorption intensity at 242 nm with respect to time. From the above plot, the pseudo first order reaction rate constant was calculated as k = 1.97 × 10⁻².

From this result, it has been shown that the pseudo first order reaction rate constant can be obtained from the change in UV spectrum of the compound, and can be useful for verification of the influence of the reductive cleavage reaction by the structure of the compound.

### Test Example 3

### Examination of Reaction between Thiophenol Model Compound and GSH Using NMR

An exchange reaction between GSH and disulfide has been extensively studied. An equilibrium constant is important in considering the exchange reaction. GSH itself is in equilibrium with reduced GSH or oxidized GS-SG, and disulfide as a linker is also in equilibrium with its oxidized form. Accordingly, determining concentration ratios of various chemical species to obtain an equilibrium constant is an index for considering reactivity between the linker disulfide and GSH. The reactivity has been investigated by various methods such as a method of obtaining an oxidation-reduction potential by electrochemical measurement and a method of obtaining a concentration ratio of each chemical species by NMR measurement.

In the present study, stability of a disulfide bond in a mixed system under various concentration conditions was examined by using NMR. According to Test Example 2, the reaction can be traced by UV, but a complicated system can be traced more by NMR, and thus, NMR measurement was used in the following.

The compounds of the formula (6), formula (7) and formula (8) each having the above-described substituent (-COOH) are designated as ArSSrA (H), ArSSrA (F), and ArSSrA (NO₂), respectively.

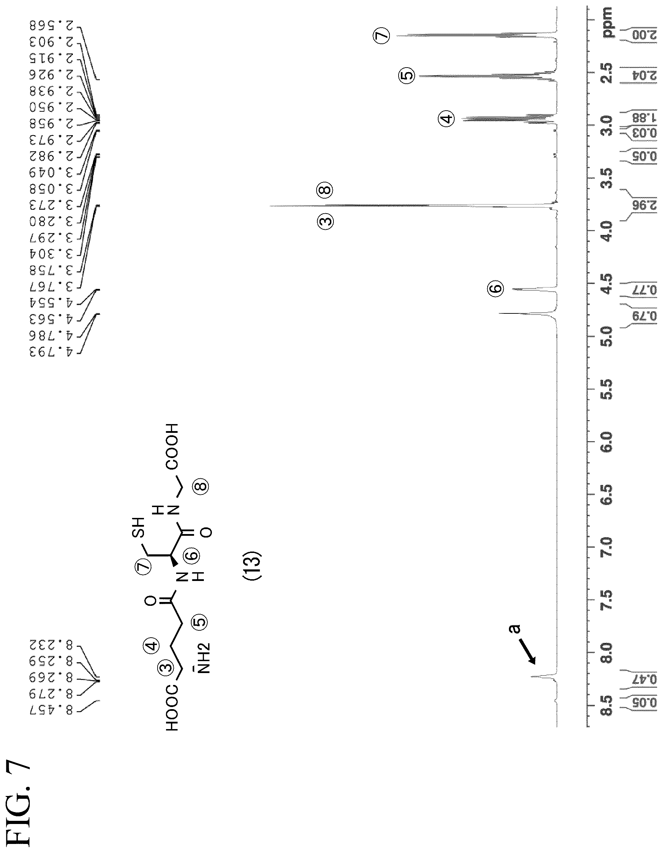

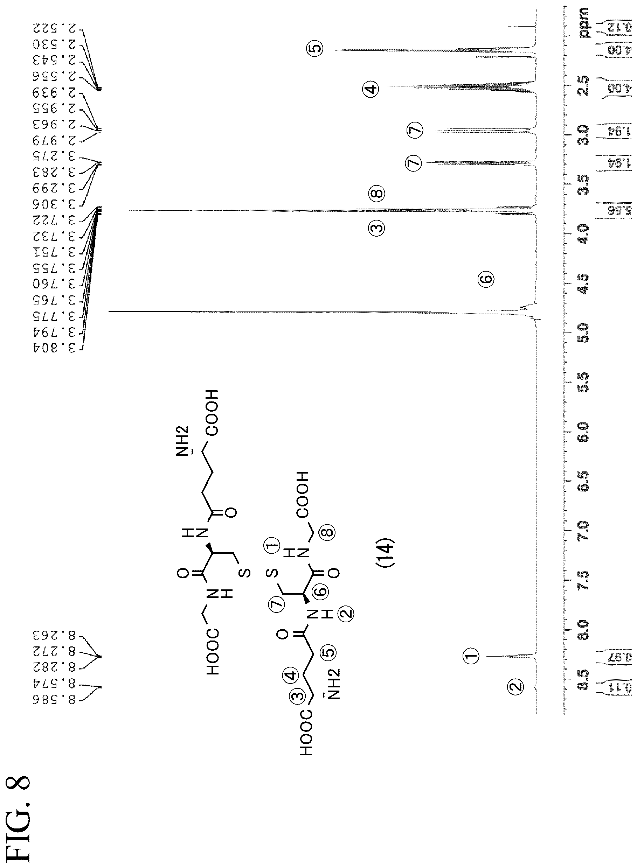

To trace a reductive cleavage reaction of a thiophenol derivative with GSH by using NMR, it is necessary to distinguish peaks of compounds present in the system, to clearly derive abundance ratios of the compounds in the system, and to conduct a detailed examination. NMR spectra of the reduced GSH are shown in FIG. 7 (formula (13) in the figure) and the oxidized GSSG shown in FIG. 8 (formula (14) in the figure) were measured.

The NMR spectra of the respective compounds are shown in FIGS. 7 and 8. Note that the peak is indicated by an in FIG. 7 is 2H of amide, and the integrated value is decreased by water-elimination (presaturation method) measurement.

When the NMR spectra of FIGS. 7 and 8 are compared, it is seen that there is a clear difference between GSH and GSSG. A particularly significant difference is a chemical shift of a proton of No. 7 at the root of the S atom. The proton of No. 7 of GSH has a peak at 2.144 ppm, while the proton of No. 7 of GSSG has two divided peaks at 3.292 ppm and 2.956 ppm. The difference in chemical shift is due to a difference in electron density between SH and SS states of the S atom, and it is considered that the reason why the peak of No. 7 of GSSG is divided is that the S atom becomes disulfide and the periphery of the S atom is sterically crowded and rotation does not occur. It was found that there was a difference between the peaks of GSH and GSSG, and thus, it was considered that the respective compound ratios in the mixed system of GSH and GSSG were able to be calculated by using integrated values.

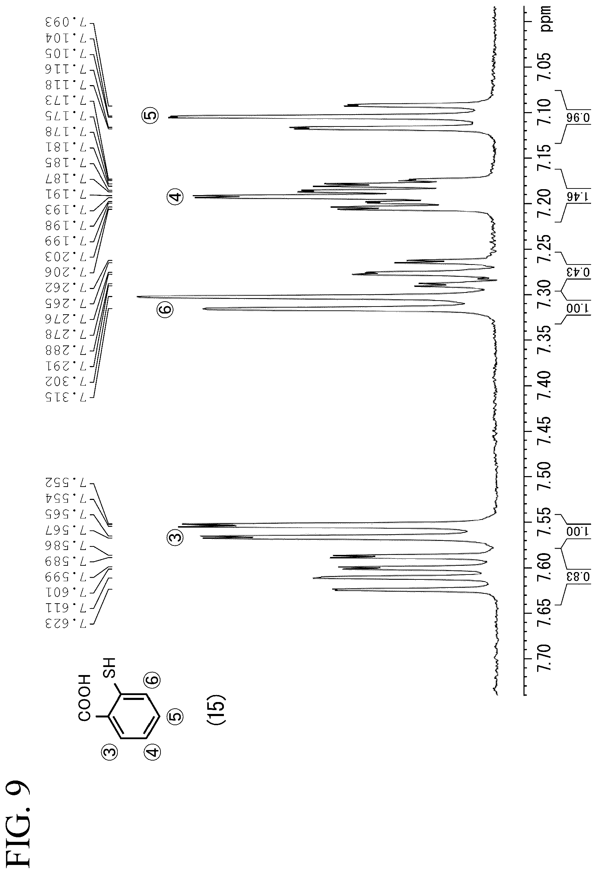

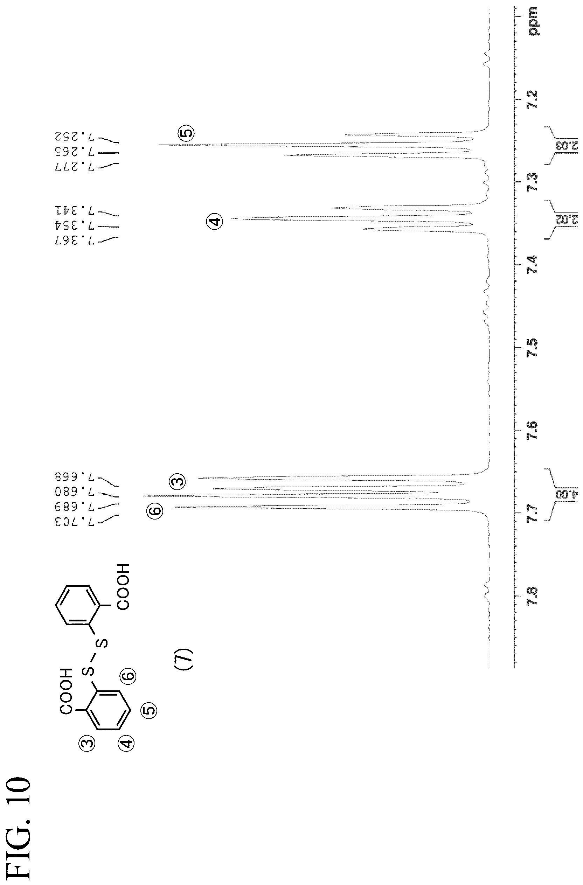

Next, FIG. 9 shows an NMR spectrum of reduced ArSH (formula (15) in the figure), and FIG. 10 shows an NMR spectrum of oxidized ArSSrA (formula (7)).

Comparison of the spectra of FIGS. 9 and 10 reveals that ArSH of the formula (15) is partially oxidized to ArSSrA of the formula (7). Thus, when the reductive cleavage reaction was actually examined in the mixed system, argon substitution was performed to prevent oxygen from entering the system as much as possible. In addition, it was found from FIG. 9 that there was a difference between the peaks of ArSH of the formula (15) and ArSSrA of the formula (7) similar to the system of GSH and GSSG, and thus, it was considered that the ratio of each compound in the mixed system could be calculated from a comparison of the integrated values of the peaks.

### Test Example 4

### Examination of Reductive Cleavage Reaction in Mixed System

A parallel relationship between ArSSrA (H) and GSH (left side) and between ArSH (H) and GS-SG (right side) is shown in a formula (16).

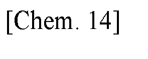

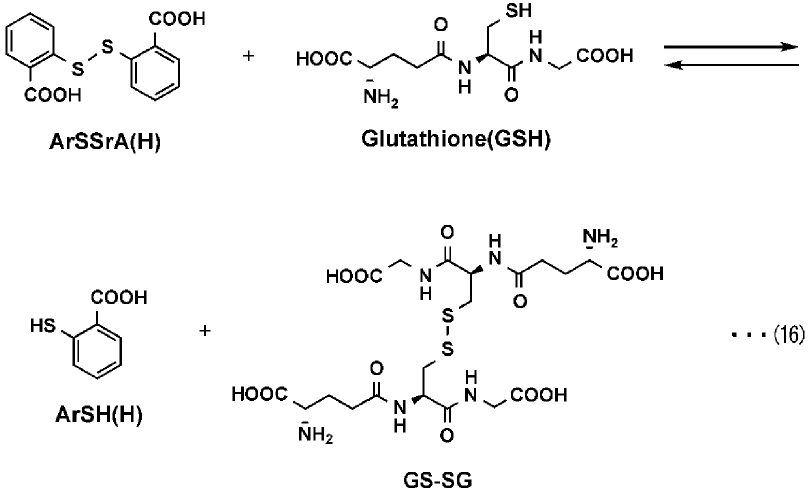

As NMR spectrum measurement conditions, GSH of the formula (13) was added to ArSSrA (H) of the formula (7) in a 50 mM phosphate buffer to adjust a solution of ArSSrA (H)/GSH = 4.4 mM/4.5 mM, and the measurement by NMR was carried out. A double tube (SHIGEMI Co., Ltd., Model SP-405) was used and a TMS-containing CDCl3 solution (LOCK solvent) was placed in the outside to adjust an NMR tube.

In the NMR measurement, the water elimination (presaturation method) measurement was performed with reference to Casi, G. et al., J. Controlled Release. 2012, 161, 121, 422 to 428, and the like.

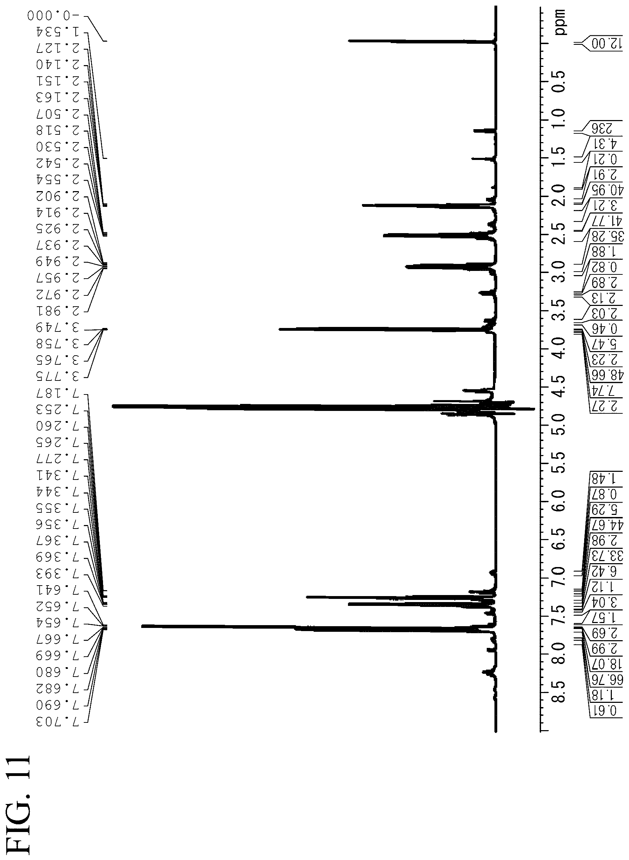

FIG. 11 shows the overall NMR spectrum 25 minutes after the NMR tube was adjusted.

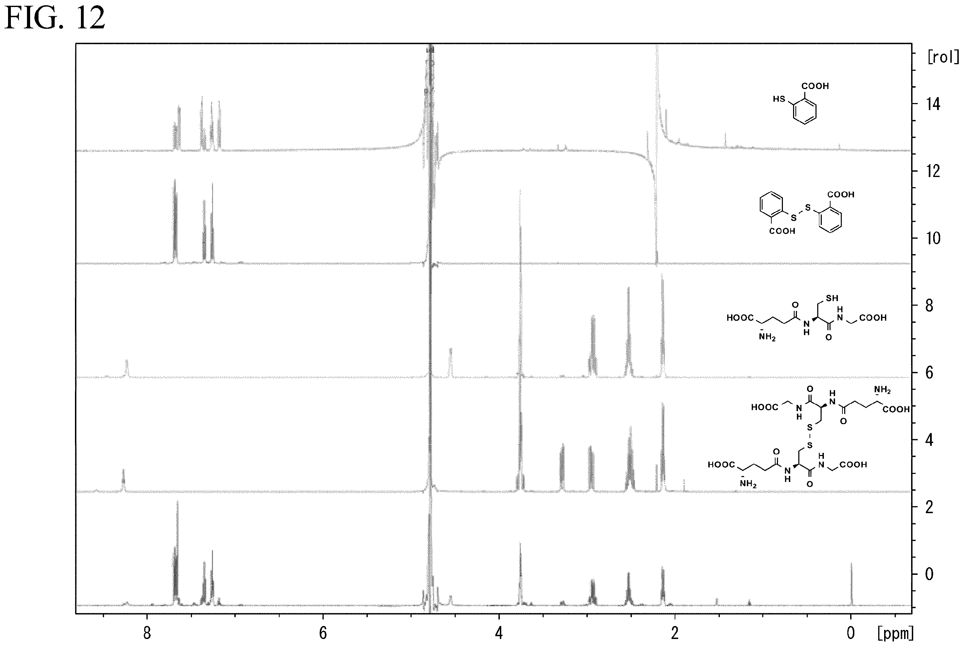

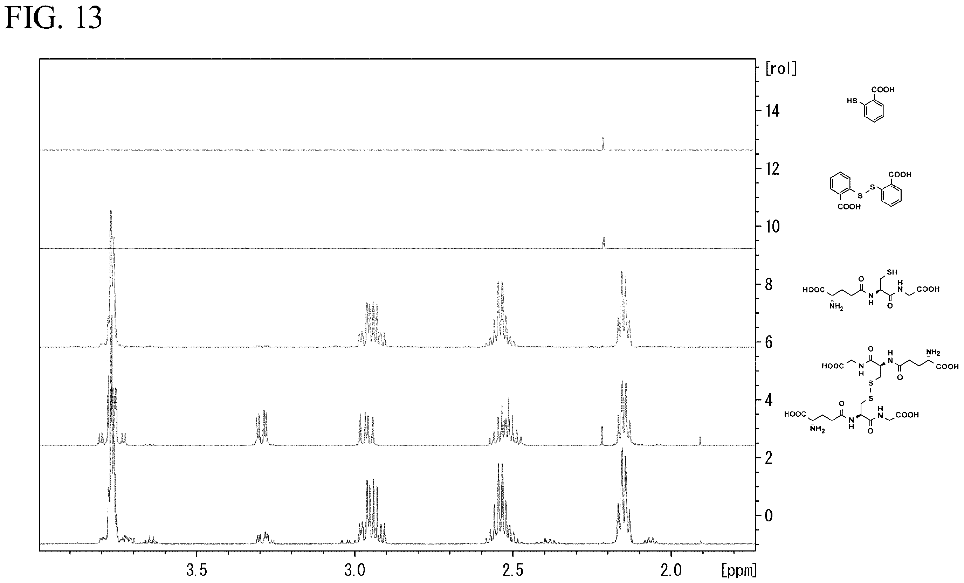

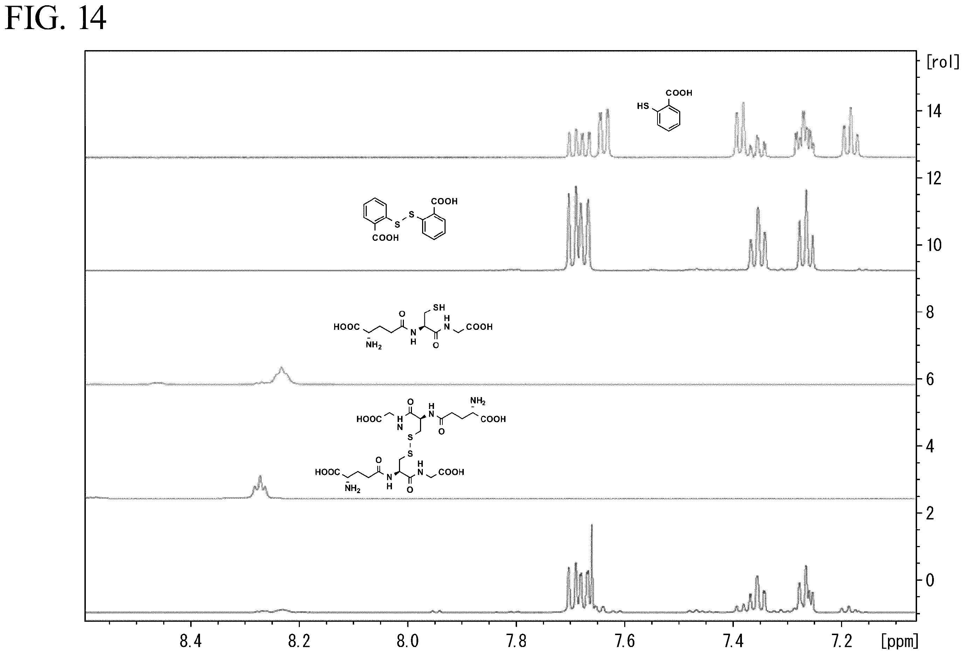

Next, for assignment and detailed analysis of each compound in the mixed system spectrum after 25 minutes shown above, FIG. 12 shows an overall view, FIG. 13 shows a partially enlarged view at about 2 to 4 ppm, and FIG. 14 shows a partially enlarged view at about 7.2 to 8.4 ppm for comparison with the NMR spectra of the following chemical species considered to be present in the mixed system according to the reaction formula shown above, ArSH (H) of the formula (15), ArSSrA (H) of the formula (7), GSH of the formula (13), and GS-SG of the formula (14). Note that in each of FIGS. 12 to 14, the spectrum at the bottom with no chemical formula is the spectrum of the mixed system.

An abundance ratio of each compound can be derived from an initial concentration added and comparison between the integrated values of the obtained spectra, and it has been found that hetero disulfide represented by the following formula (17) (hereinafter, also referred to as (H) ARs-SG) is present in the system as an intermediate.

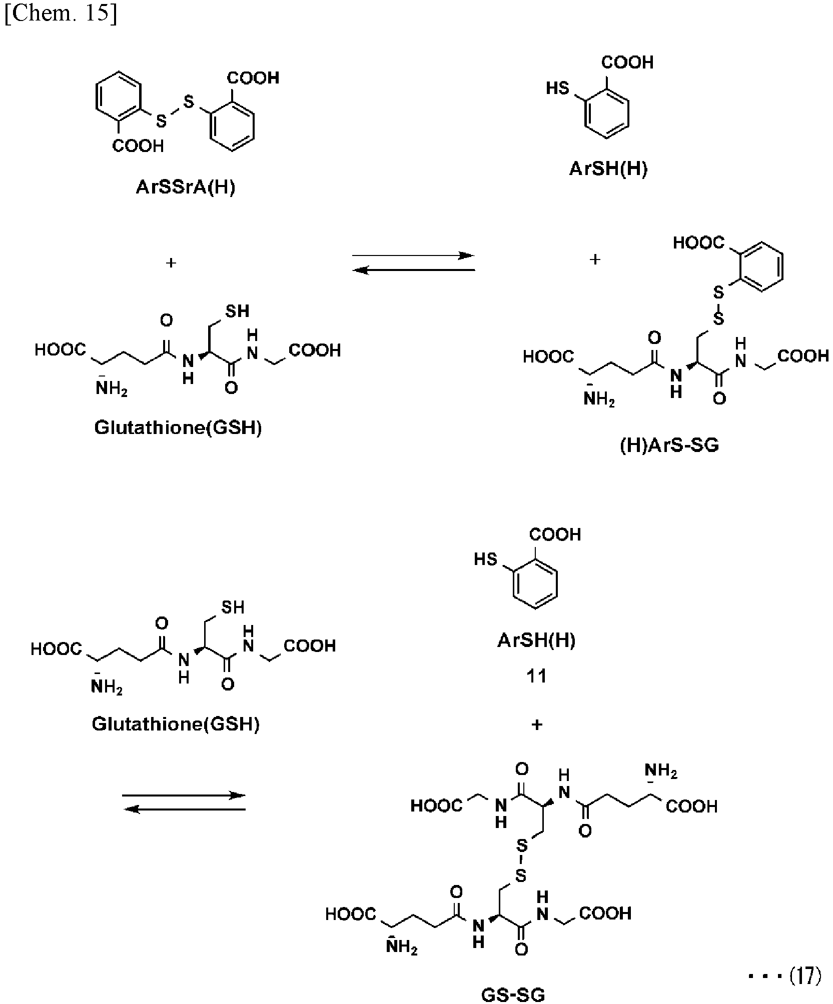

It has been found that the abundance ratio of each compound after 25 minutes, 100 minutes, and 1 day from adjustment was as shown in the following Table 1, by comparing the integrated values of the obtained spectra. (Initial concentration ArSSrA (H)/GSH = 4.4 mM/4.5 mM)

**[Table 1]**

| R = COOH, H | ArS-SrA | ArSH | ArS-SG | ArS-SG | GS-SG | GSH |
|---|---|---|---|---|---|---|
| 0 min. | 100% | 0 | 0 | 0 | 0 | 100% |
| 25 min. | 83 | 13 | 3.8 | 7.2 | 19 | 74 |
| 100 min. | 62 | 26 | 11 | 20 | 32 | 48 |
| 1 day | 57 | 12 | 31 | 57 | 32 | 11 |

From the above table, it can be seen that reduced GSH is consumed and the amount thereof decreases with time, whereas the amount of oxidized GS-SG does not change greatly and the amount of hetero disulfide (H) ArS-SG increases. In addition, it can be seen that the change of ArSH (H) generated by cleavage is complicated, and after 100 minutes, the amount of ArSH (H) is twice as large as that after 25 minutes, whereas after 1 day, ArSH (H) is changed to (H) ArS-SG and ArS-SrA (H). From the above results, it was found that ArS-SrA (H) was surely cleaved by GSH, and thus, it was suggested that drug release was possible in DDS using ArSSrA (H) as a linker.

### Test Example 5

### Examination of Cleavage Reaction under Normal Cell Model Condition

ArS-SrA (H) of the formula (7) was administered to a cell model of a GSH concentration assuming a realistic intracellular drug concentration to examine the cleavage reaction of the linker compound.

At an ArS-SrA (H) concentration (225 µM), the GSH concentration of a normal cell was approximated to 450 µM, and the same measurement was performed. To prevent a peak of the aromatic region having a low concentration from being overlapped by the peak of CHCl₃ contained in CDCl₃, the measurement was performed with D₂O in outside the double tube. The overall compound concentration was low and the peak ratio of water was high, and thus, the number of times of integration was increased for measurement to distinguish between the peaks of the compounds and the peak of the baseline. The analysis results are shown in Table 2.

**[Table 2]**

| R = COOH, H | ArS-SrA | ArSH | ArS-SG | ArS-SG | GS-SG | GSH |
|---|---|---|---|---|---|---|
| 0 min. | 100% | 0 | 0 | 0 | 0 | 100% |
| 100 min. | 89 | 8.2 | 2 | 1.8 | 10 | 88 |
| 1 day | 84 | 3.3 | 24 | 21 | 62 | 18 |

From the above table, it was found that under the model GSH concentration condition of a normal cell, 18% of GSH remained after 1 day and 82% thereof was consumed, whereas 84% of linker model disulfide ArS-SrA (H) remained after 1 day and only about 16% thereof was cleaved, and much of ArS-SrA (H) remained without being consumed.

### Test Example 6

### Examination of Cleavage Reaction under Cancer Cell Model Condition

At a concentration of ArS-SrA (H) of the formula (7) (225 µM) assuming a realistic intracellular drug concentration, a GSH concentration of a cancer cell was approximated to 20 mM, and the same measurement was performed.

To prevent a peak of the aromatic region having a low concentration from being overlapped by the peak of CHCl3 contained in CDCl3, the measurement was performed with D2O in the outside the double tube. The overall compound concentration was low and the peak ratio of water was high, and thus, the number of times of integration was increased for measurement to distinguish between the peaks of the compounds and the peak of the baseline. The analysis results are shown in Table 3.

**Table 3**

| R = COOH, H | ArS-SrA | ArSH | ArS-SG | ArS-SG | GS-SG | GSH |
|---|---|---|---|---|---|---|
| 0 min. | 100% | 0 | 0 | 0 | 0 | 100% |
| 100 min. | 19 | 82 | 0 | 0 | 3.4 | 97 |
| 1 day | 0 | 100 | 0 | 0 | 8.8 | 91 |

From the above results, it has been found that 19% of ArS-SrA (H) remained and 82% thereof was cleaved after 100 minutes, and 0% thereof remained and 100% thereof was cleaved after 1 day.

When this result is compared with the result at the GSH concentration in a normal cell in Test Example 5, there is a clear difference, and it can be seen that at the GSH concentration assuming a cancer cell, most of (H) ArS-SG and ArSSrA (H) is cleaved by GSH and converted to ArSH (H). From the above results, it has been found that ArS-SrA (H) at a realistic intracellular drug concentration can be expected as a linker that is cleaved in response to the normal cell GSH concentration and the cancer cell GSH concentration.

### Test Example 7

### Examination of Reductive Cleavage Reaction of F-Substituted Product

Next, ArSSrA (F) shown in the following formula (18) was used under the same conditions as in Test Example 5 to examine whether a cleavage reaction occurs as in the case of ArSSrA (H). The analysis results are shown in Table 4. As shown in Table 4, it was found that about 40% of ArSSrA (F) remained without being cleaved after 1 day.

It was confirmed that it was also cleaved in the same manner as in ArSSrA (H).

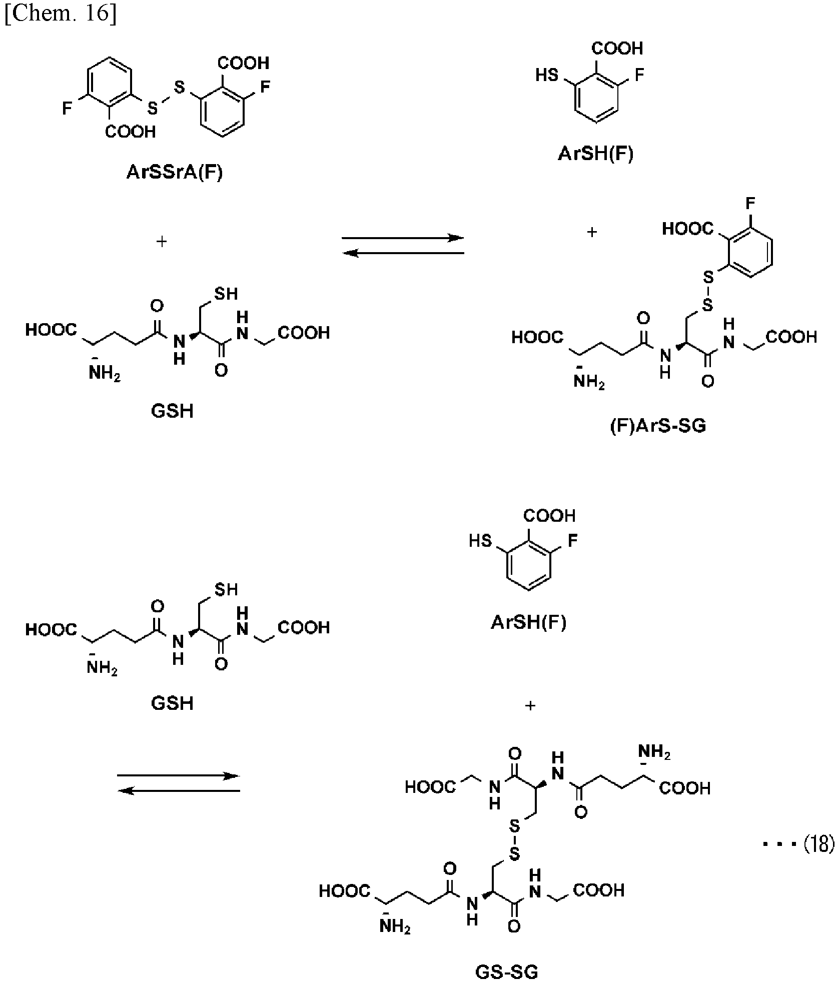

**Table 4**

| R = COOH, F | ArS-SrA(F) | ArSH(F) | ArS-SG (F) | ArS-SG | GS-SG | GSH |
|---|---|---|---|---|---|---|
| 0 min. | 100% | 0 | 0 | 0 | 0 | 100% |
| 100 min. | 42 | 38 | 20 | 39 | 9 | 42 |
| 1 day | 43 | 36 | 21 | 41 | 26 | 8 |

### Test Example 8

### Examination of Reductive Cleavage Reaction of NO2-Substituted Product

ArSSrA (NO2) represented in the following formula (19) was used under the same conditions as in Test Example 5 to examine whether a cleavage reaction occurs as in the case of ArSSrA (H). The analysis results are shown in Table 5. As shown in Table 5, it was found that about 30% of ArSSrA (NO2) remained without being cleaved after 1 day.

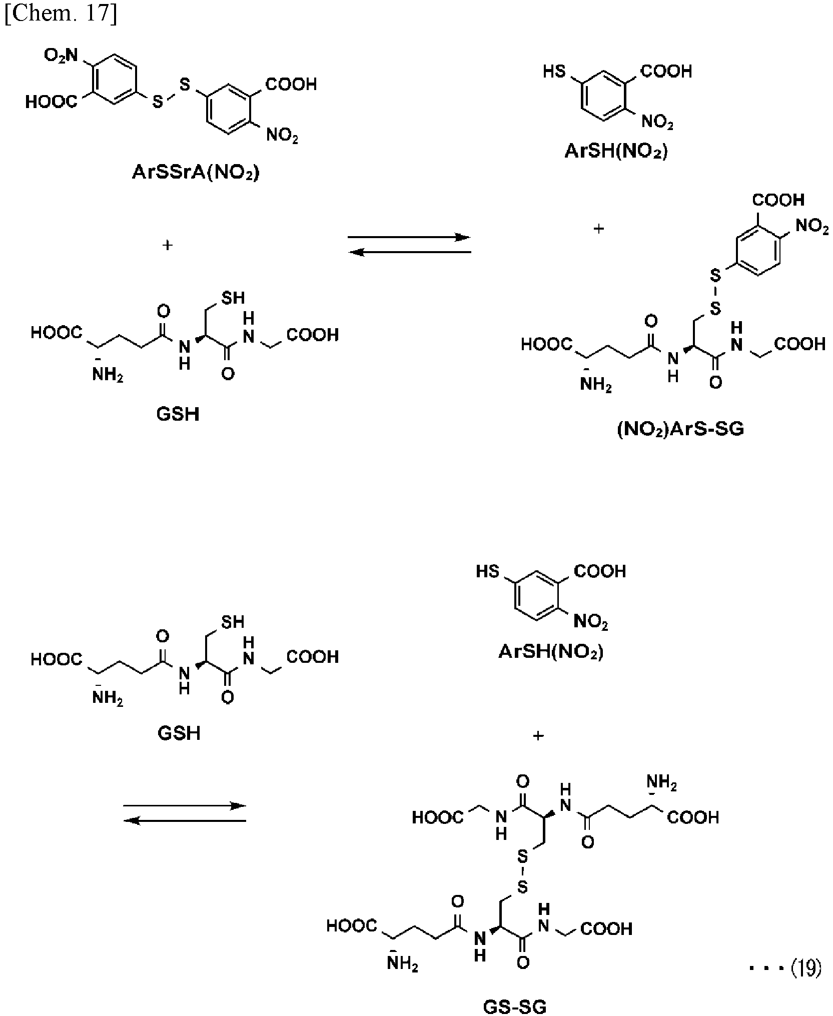

**Table 5**

| R = COOH, (NO₂) | ArS-SrA (NO₂) | ArSH (NO₂) | ArS-SG (NO₂) | ArS-SG | GS-SG | GSH |
|---|---|---|---|---|---|---|
| 0 min. | 100% | 0 | 0 | 0 | 0 | 100% |
| 100 min | 29 | 51 | 20 | 39 | 9 | 30 |
| 1 day | 30 | 48 | 22 | 45 | 26 | 4 |

### Test Example 9

### Reductive Cleavage Reaction by GSH: Influence of Different GSH concentrations and Substituents

ArSSrA (F) and ArSSrA (NO₂) were subjected to the same test as in Test Example 6 to examine their cleavage reactions in the normal cell model and the cancer cell model. The results of ArSSrA (F) and ArSSrA (NO₂) are shown in Table 6 and Table 7, respectively. As shown in Table 6 and Table 7, it was found that about 90% of each of ArSSrA (F) and ArSSrA (NO₂) was cleaved after 100 minutes and all thereof was cleaved after 1 day, and it was found that ArSSrA (F) and ArSSrA (NO₂) can be expected as a linker that is cleaved in response to the GSH concentration in a normal cell and the GSH concentration in a cancer cell.

**Table 6**

| R = COOH, F | ArS-SrA(F) | ArSH(F) | ArS-SG(F) | ArS-SG | GS-SG | GSH |
|---|---|---|---|---|---|---|
| 0 min. | 100% | 0 | 0 | 0 | 0 | 100% |
| 100 min. | 8 | 92 | 0 | 0 | 3.8 | 96 |
| 1 day | 0 | 100 | 0 | 0 | 9.5 | 90 |

**Table 7**

| R = COOH, (NO₂) | ArS-SrA (NO₂) | ArSH (NO₂) | ArS-SG (NO₂) | ArS-SG | GS-SG | GSH |
|---|---|---|---|---|---|---|
| 0 min. | 100% | 0 | 0 | 0 | 0 | 100% |
| 100 min | 5 | 95 | 0 | 0 | 4 | 95 |
| 1 day | 0 | 100 | 0 | 0 | 10 | 90 |

### Test Example 10

### Selective Drug Release in Cancer Cell Characteristic Cytoplasmic Environment

Cultured cells were used to test whether cleavage by the linker compound of the present embodiment specifically occurs under the condition of a cancer cell.

First, an Arg₈-Dbs-linker-FAM compound was prepared in which FAM (formula (20)) and Dbs (formula (21))-Arg₈ were bonded to the two Rs of the linker of the formula (1) (formula (22)).

Args functions as a cell membrane permeable peptide (arginine linker), and has an action of incorporating a bound compound into cytoplasm.

Each of FAM and Dbs develops color by fluorescence, but a compound to which FAM-Dbs is bound hardly develops color. Thus, in a case where the linker compound is cleaved, FAM and DBS are separated and emit light, and thus, it is possible to detect distribution of FAM and DBS in a cell.

Accordingly, when the fluorescence emission of the Arg₈-Dbs-linker-FAM compound is investigated, it is possible to detect whether the compound is taken up into the cytoplasm or cleaved in the cell, and the distribution of compounds generated by the cleavage in the cell.

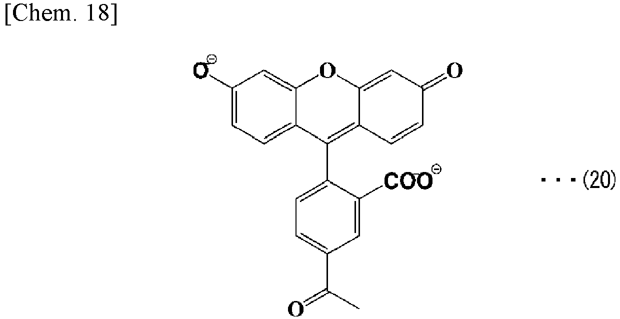

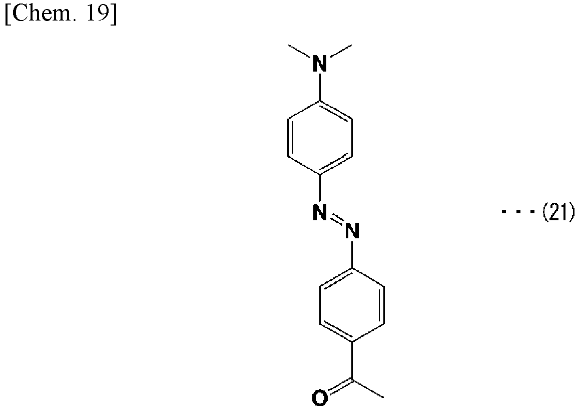

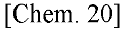

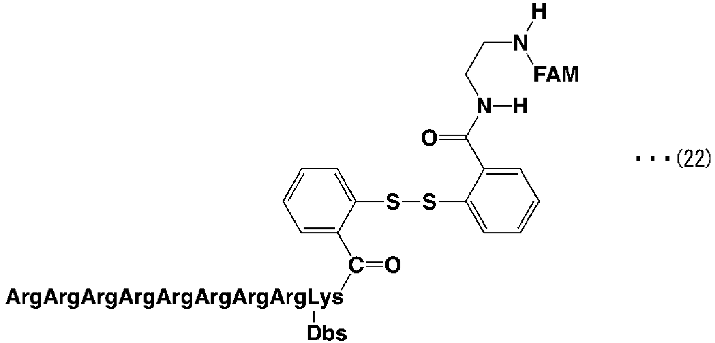

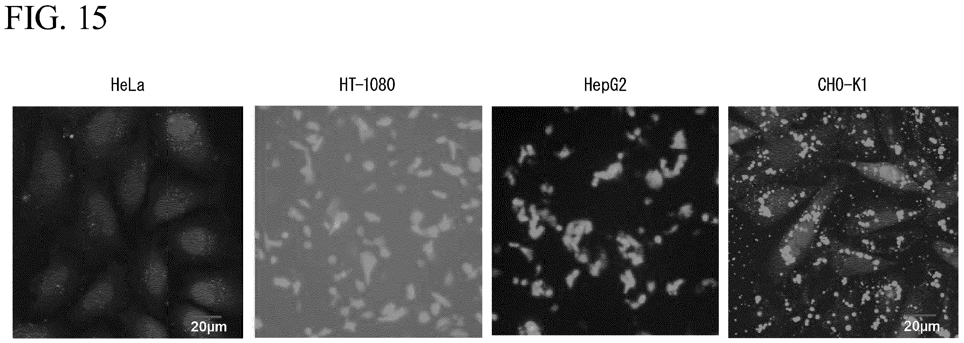

FIG. 15 shows a view obtained by incubating various cells with the Arg₈-Dbs-linker-FAM compound (formula (22)) (25 µM) in an F-12 (-) state for 30 minutes, and observing fluorescence development with a confocal microscope. The various cells are HeLa (human cervical epithelial carcinoma cell), HT-1080 (human fibroblast sarcoma cell), HepG2 (human hepatoma cell), and CHO-K1 (Chinese hamster ovary-derived cell), respectively.

In all the cases, strong fluorescence development was observed in the cell, indicating that the compound was taken up into the cell by the cell membrane permeable peptide (arginine linker). Furthermore, from the strong fluorescence development, it was confirmed that Dbs and FAM were separated from the compound of the formula (22), that is, the disulfide bond of the linker compound was cleaved.

From this result, it has been shown that the compound bound by the linker compound of the present embodiment can be introduced into a cell by the cell membrane permeable peptide, and the linker compound can be cleaved only in a cancer cell having the glutathione concentration of a certain level or more.

### Industrial Applicability

According to the linker compound and the complex compound containing the same of the present invention, the glutathione concentration at which the disulfide bond dissociates can be designed by molecular design, a cancer cell can be accurately identified, and the linker compound is stable outside a target cell and can effectively release the drug compound in the target cell. Accordingly, the linker compound and the complex compound containing the same of the present invention can be widely applied to an antibody drug using an antibody-drug complex and a drug using another complex, and can be used for cancer treatment with fewer side effects.

## Claims

1. A linker compound for use in a complex compound containing a drug compound, the linker compound having
a structure represented by the following formula (1): 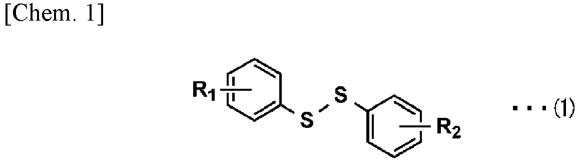
where R₁ and R₂ are each a compound group including hydrogen, carbon, oxygen, nitrogen, or a halogen element, or a derivative thereof.

2. The linker compound according to claim 1, wherein, in the formula (1), R₁ and R₂ are each a halogen element, a hydrocarbon group, an oxide group, or a nitride group.

3. The linker compound according to claim 1 or 2, wherein, in the formula (1), R₁ and R₂ are each a halogen element, a methyl group, a t-butyl group, a methoxy group, a nitro group, a carboxyl group, a cyano group, or a dimethylamino group.

4. The linker compound according to any one of claims 1 to 3, wherein, in the formula (1), R₁ and R₂ are each a nitro group, fluorine, or a carboxyl group.

5. The linker compound according to any one of claims 1 to 4, wherein, in the formula (1), the S-S bond is cleaved at a glutathione concentration of 10 to 200 mM.

6. The linker compound according to any one of claims 1 to 5, wherein the complex compound is an antibody-drug complex compound in which the drug compound is complexed with a drug compound-transporting peptide via the linker compound.

7. The linker compound according to claim 6, wherein the drug compound-transporting peptide is selected from the group consisting of an antibody and a cell membrane permeable peptide.

8. A complex compound comprising the linker compound described in any one of claims 1 to 7.

9. The complex compound according to claim 8, wherein the complex compound is an antibody-drug complex compound in which the drug compound is complexed with the antibody via the linker compound.

10. The complex compound according to claim 8 or 9, wherein the drug compound is an apoptosis fusion peptide.

11. The complex compound according to any one of claims 8 to 10, wherein the drug compound is a nucleic acid.

12. The complex compound according to any one of claims 8 to 11, wherein the complex compound is used against a cancer cell.
